# FASCICULE DE BREVET EUROPEEN

(11) **EP 0 819 009 B1**
(45) Date de publication et mention de la délivrance du brevet: **10.04.2002**
(21) Numéro de dépôt: 97902422.1
(22) Date de dépôt: 31.01.1997
(51) Int. Cl.: A61K 38/24

(54) **MEDICAMENTS POUR LE DECLENCHEMENT DE L'OVULATION CONTENANT L'HORMONE LUTEINISANTE**
OVULATION AUSLÖSENDE ARZNEISTOFFE ENTHALTEND LUTEINISIERENDES HORMON
OVULATION TRIGGERING DRUGS COMPRISING LUTEINISING HORMONE

(30) Priorité: 02.02.1996 FR 9601303
(43) Date de publication de la demande: 21.01.1998
(73) Titulaire: Applied Research Systems ARS Holding N.V., Curacao (AN)
(72) Inventeur: Emperaire, Jean-Claude, 33000 Bordeaux (FR)
(74) Mandataire: Peaucelle, Chantal
(86) Numéro de dépôt international: FR9700198
(87) Numéro de publication internationale: WO9727868

(56) Documents cités:
- EP-A- 0 119 168
- EP-A- 0 193 277
- WO-A-93/13799
- FR-A- 2 581 544

## Description

L'invention a pour objet des médicaments pour le déclenchement de l'ovulation.

On sait que l'ovulation est déclenchée physiologiquement par une décharge de LH, qui intervient au milieu du cycle et provoque l'ovulation à partir du follicule mûr ; une décharge de FSH de moindre amplitude intervient de manière synchrone, agissant probablement en synergie avec LH.

En thérapeutique, même si un déclenchement spontané de l'ovulation est toujours possible, il est préférable de provoquer le déclenchement de l'ovulation, pour des raisons d'efficacité et de programmation.

Depuis les années 30, on utilise le plus généralement, à cet effet, l'hormone chorionique gonadotrope, ou HCG, extraite de l'urine des femmes enceintes, qui possède une activité biologique de type LH.

Depuis cette époque, le déclenchement de l'ovulation au cours des cycles stimulés par les anti-oestrogènes, ou les HMG, est assuré par une injection unique de 5000 ou de 10000 U.I. de HCG.

L'HCG présente toutefois sa propre spécificité par rapport à la LH, donnant lieu à des réactions différentes avec les récepteurs, et lui conférant une demi-vie prolongée par rapport à la LH.

L'HCG présente l'avantage d'être efficace pour le déclenchement de l'ovulation et de faible prix de revient.

Mais elle présente des inconvénients potentiels très importants. En dehors de la nécessité de disposer de collections d'urine suffisantes pour extraire des quantités commercialisables d'HCG, deux risques majeurs peuvent être observés, à savoir un effet d'hyperstimulation ovarienne, plus ou moins redoutable lorsque l'administration d'HCG intervient sur une stimulation folliculaire exagérée et des grossesses multiples, ce qui constitue un corollaire de la complication précédente.

Ces deux inconvénients entachent de manière significative l'utilisation de l'HCG en thérapeutique, et rendent souhaitable l'utilisation de LH pour déclencher l'ovulation.

L'utilisation de LH endogène a été envisagée. A de très rares exceptions près, les patientes stimulées sur le plan ovulatoire possèdent une réserve hypophysaire de LH, qui peut être mobilisée par l'utilisation du GnRH lui-même, ou de l'un de ses agonistes tels que disponibles dans le commerce. L'administration d'un agoniste entraîne une décharge très importante de LH, tout à fait comparable au pic physiologique pré-ovulatoire, mais la durée de cette décharge dépasse rarement 24 h, alors que le pic physiologique s'étale sur environ 48h.

Ce mode de déclenchement ovulatoire par la LH endogène permet d'obtenir une quasi disparition du risque d'hyperstimulation ovarienne, résultant d'une activité biologique moindre de la LH et de sa demi-vie plus courte que celle de l'HCG. Sa demi-vie de dilution est en effet d'une heure environ et sa demi-vie d'élimination de 24h environ,soit environ dix fois moins que celle de l'HCG. De même, il apparaît que le risque de grossesse multiple est plus faible lorsqu'on utilise cette méthode.

Enfin, l'administration d'un agoniste du GnRH mobilise également la FSH hypophysaire de manière synchrone avec la décharge de LH, en reproduisant au plus près le pic physiologique.

Cependant, la briéveté de la décharge de la LH ainsi provoquée pour déclencher l'ovulation n'est pas appropriée chez un certain nombre de femmes. Chaque femme possède en effet un profil de pic de LH spécifique et, d'une manière générale, une élévation de LH supérieure à 24 h est nécessaire pour déclencher une ovulation dans de bonnes conditions. Il s'ensuit que le déclenchement de l'ovulation par la LH endogène mobilisée par un agoniste de la GnRH peut être un facteur de phase lutéale de bonne qualité, avec des chances conceptionnelles satisfaisantes, mais aussi de phase lutéale courte (d'une durée inférieure ou égale à 8 jours) ou inadéquate (de longueur normale mais avec une progestéronémie inférieure à 8 ng/ml).

Pour remédier à ces inconvénients, on a proposé d'utiliser de la LH exogène, d'origine hypophysaire humaine. Cependant, pour des raisons évidentes de disponibilité, les expérimentations avec un tel produit n'ont pu être prolongées longtemps. L'obtention de LH sous forme recombinante a permis de poursuivre ce type d'expérimentations, mais compte tenu de la demi-vie de la LH, le déclenchement de l'ovulation par la LH recombinante nécessite une injection de doses importantes. Des doses plus faibles sont susceptibles d'induire un pic plasmatique de LH relativement bref par rapport au pic physiologique, risquant d'entraîner les mêmes problèmes qu'avec la LH endogène.

Les injections doivent être alors répétées, ce qui pose un double problème d'acceptabilité pour la patiente, et de coût, le prix de revient de la LH recombinante étant élevé.

Les travaux de l'inventeur dans ce domaine ont montré qu'il est possible de remédier aux problèmes évoqués ci-dessus en administrant la LH sous une forme galénique assurant une libération permettant de reproduire au plus près le pic physiologique, de 48h environ.

L'invention a donc pour but de fournir de nouvelles formes d'administration de médicaments à base de LH pour le déclenchement de l'ovulation.

Les médicaments pour le déclenchement de l'ovulation selon l'invention sont caractérisés en ce qu'ils comportent de la LH sous une forme d'administration assurant une augmentation du taux plasmatique de LH pendant 40 à 60 h environ.

L'utilisation de LH sous une telle forme d'administration provoque un processus ovulatoire satisfaisant.

De manière avantageuse, la LH peut être utilisée en association avec de la FSH, ce qui permet de reproduire au plus près le processus physiologique et sans doute de diminuer la quantité de LH nécessaire pour déclencher l'ovulation.

Des proportions appropriées correspondent à un rapport de LH à FSH d'environ 5.

On a recours aux formes endogènes de ces hormones ou à leurs sous-unités α β, ou encore à des formes recombinantes de ces hormones ou de leurs sous-unités, tels que produits par génie génétique. Il est également possible d'utiliser des analogues thérapeutiques de la LH ou la FSH, ou des agonistes de LH et de FSH, peptidiques ou non peptidiques, éventuellement à durée de vie prolongée. Des composés de ce type peuvent être produits par génie génétique, ou par voie de synthèse et sont décrits, par exemple, par Boime et al dans "In GnRH, GnRH analogs, gonadotropins and gonadal peptides", P. Bouchard, A. Caraty, HJT COENINGH BENNINK and SN PAVLOV Edtrs, The Parthenon Publishing Group, London 1993, p 347-356.

Les formes d'administration correspondent à des particules ayant une taille de 1 à 250µ environ, voire inférieure.

Selon un mode de réalisation de l'invention, ces particules sont à base d'un concentré de LH et le cas échéant de FSH.

Des particules préférées de ce type sont constituées par des lyophilisats, le cas échéant stabilisés au moyen de sucrose et/ou de sels d'acides dicarboxyliques.

Selon un autre mode de réalisation de l'invention, il s'agit de particules comprenant une enveloppe solide à l'intérieur de laquelle se trouve la LH, associée le cas échéant à de la FSH et à des excipients pharmaceutiques.

L'enveloppe contrôle alors éventuellement la libération de principe actif.

Des matériaux appropriés pour former l'enveloppe comprennent des polymères biodégradables. Parmi ces polymères, on citera l'acide polylactique ou un copolymère d'acide lactique et d'acide glycolique.

Selon une autre variante, les particules sont constituées par un réseau continu de matériau support dans lequel est dispersé le principe actif.

L'administration de ces différentes formes est réalisée de manière à effectuer une injection ou un implant sous-cutané ou intramusculaire.

La dose de LH injectée pour obtenir un processus ovulatoire satisfaisant sous cette forme doit être comprise entre 10 000 et 25 000 U.I. environ.

Les études de toxicologie effectuées montrent l'innocuité des médicaments de l'invention.

D'autres caractéristiques et avantages de l'invention sont donnés dans les exemples qui suivent :

### Exemple 1 : Préparation injectable

On prépare des flacons contenant des microsphères de LH renfermant les ingrédients suivants:
LH: 3 mg
excipient: polymère de dl-lactide-coglycolide: environ 170 mg
mannitol: 85 mg
carboxyméthylcellulose sodique: 30 mg
polysorbate 80: 2 mg
Comme milieu de mise en suspension, pour une ampoule, on utilise:
mannitol: 16 mg
eau pour préparation injectable: q.s.p. 2g

Le copolymère de lactide-glycolide est un polyester synthétique biocompatible et biodégradable, tel qu'utilisé en médecine humaine, notamment pour la fabrication de fils chirurgicaux résorbables.

Obtention des microsphères: On opère avantageusement comme décrit par Ogawa et al dans Chem. Pharm. Bull, 36 (3) 1095-1103, 1988.

Les microsphères sont obtenues par transfert des macromolécules d'un état solvatisé à une interphase, à savoir la phase coacervée. Le produit de coacervation esr ensuite transformé en gel contenant les particules de principe actif, puis solidifié.

On procède à l'addition, lentement et sous agitation, d'une dispersion de principe actif dans une solution de copolymère de lactide-glycolide. On diminue progressivement la solubilité du copolymère en ajoutant un non-solvant. Le copolymère se coacerve alors lentement autour des particules de LH en suspension. Les microsphères sont ensuite durcies par immersion, séparées par filtration, lavées, séchées et analysées pour déterminer leur teneur en LH.

On réalise des conditionnements appropriés en ajoutant aux microsphères les autres ingrédients nécessaires pour réaliser une injection ou un implant sous-cutané ou intramusculaire.

### Exemple 2 : Etudes de toxicologie

Aucun signe de toxicité n'est apparu pour des doses testées jusqu'à 200 000 U.I./kg par voie sous-cutanée et intraveineuse, que ce soit en étude de toxicité aigüe pour une dose unique ou de toxicité chronique. Cette dose représente au moins 1000 fois la dose qu'il est prévu d'utiliser en clinique.

Les mesures de toxicité aigües donnent une DL50 par voie intrapéritonéale de l'ordre de 100 mg chez le rat. La DL50 n'a pu être déterminée à des doses de 200 000 fois (souris) la dose thérapeutique habituelle.

L'étude de la toxicité chronique chez le rat et le singe, à des doses de 2, 20, 200 µg/kg de microparticules, par voie sous-cutanée, tous les jours pendant une période de 6 mois, montre que les effets observés sont essentiellement en rapport avec l'activité de la LH, ou de la LH associée à de la FSH.

## Revendications

1. Médicaments pour le déclenchement de l'ovulation, **caractérisés en ce qu'**ils comportent de la LH sous une forme d'administration assurant une augmentation du taux plasmatique de LH pendant 40 à 60 h environ.

2. Médicaments selon la revendication 1, **caractérisés en ce qu'**ils renferment en outre de la FSH.

3. Médicaments selon l'une des revendications 1 ou 2, **caractérisés en ce que** la LH, et le cas échéant la FSH, correspondent aux hormones endogènes, à leurs sous-unités, à des formes recombinantes de ces hormones ou à des analogues ou des agonistes, tels que produits par génie génétique ou par voie de synthèse.

4. Médicaments selon l'une des revendications 1 à 3, **caractérisés en ce qu'**ils se présentent sous forme de particules de moins de 1 à 250 µ.

5. Médicaments selon la revendication 4, **caractérisés en ce que** lesdites particules sont à base d'un concentré de LH et, le cas échéant, de FSH.

6. Médicaments selon la revendication 5, **caractérisés en ce qu'**ils sont stabilisés à l'aide de sucrose et/ou de sels d'acides dicarboxyliques.

7. Médicaments selon la revendication 4, **caractérisés en ce que** lesdites particules comprennent une enveloppe solide à l'intérieur de laquelle se trouve la LH, le cas échéant associée à la FSH, et à des excipients pharmaceutiques.

8. Médicaments selon la revendication 7, **caractérisés en ce que** l'enveloppe est élaborée à partir de polymères biodégradables, tels que l'acide polylactique ou un copolymère d'acide lactique et d'acide glycolique.

9. Médicaments selon la revendication 8, **caractérisés en ce que** lesdites particules sont constituées par un réseau continu de matériau support dans lequel est dispersée la LH, le cas échéant associée à la FSH.

## Claims

1. Medicaments to trigger ovulation, **characterized in that** they comprise LH in a form of administration ensuring an increase in the plasmatic level of LH for about 40 to 60 hours.

2. Medicaments according to claim 1, **characterized in that** they contain in addition FSH.

3. Medicaments according to one of claims 1 or 2, **characterized in that** LH, and if appropriate FSH, correspond to endogenous hormones, to their subunits, to recombinant forms of these hormones or to analogues or agonists, as produced by genetic engineering or synthesis.

4. Medicaments according to one of claims 1 to 3, **characterized in that** they are presented in the form of particles of less than 1 to 250 p.

5. Medicaments according to claim 4, **characterized in that** the said particles are a based on an LH concentrate and, if appropriate FSH.

6. Medicaments according to claim 5, **characterized in that** they are stabilized using sucrose and/or dicarboxylic acid salts.

7. Medicaments according to claim 4, **characterized in that** the said particles consist of a solid envelope inside which is found the LH, if appropriate combined with FSH and with pharmaceutical excipients.

8. Medicaments according to claim 7, **characterized in that** the envelope is manufactured from biodegradable polymers, such as polylactic acid or a lactic acid and glycolic acid copolymer.

9. Medicaments according to claim 8, **characterized in that** the said particles are comprised of a continuous network of support material in which the LH is dispersed, if appropriate combined with FSH.

## Patentansprüche

1. Arzneimittel zum Auslösen der Ovulation, **dadurch gekennzeichnet, daß** sie LH in einer Verabreichungsform enthalten, welche eine Erhöhung des LH-Gehalt im Plasma während etwas 40 bis 60 Stunden gewährleistet.

2. Arzneimittel nach Anspruch 1, **dadurch gekennzeichnet, daß** sie außerdem FSH enthalten.

3. Arzneimittel nach einem der Ansprüche 1 oder 2, **dadurch gekennzeichnet, daß** das LH und gegebenenfalls das FSH den endogenen Hormonen, deren Subeinheiten, rekombinanten Formen dieser Hormone oder Analogen oder Agonisten entsprechen, wie sie gentechnologisch oder synthetisch hergestellt sind.

4. Arzneimittel nach einem der Ansprüche 1 bis 3, **dadurch gekennzeichnet, daß** sie in Form von Teilchen von weniger als 1 bis 250 µm vorliegen.

5. Arzneimittel nach Anspruch 4, **dadurch gekennzeichnet, daß** die Teilchen als Basis ein Konzentrat von LH und gegebenenfalls FSH haben.

6. Arzneimittel nach Anspruch 5, **dadurch gekennzeichnet, daß** sie mit Hilfe von Sukrose und/oder Dikarbonsäuresalzen stabilisiert sind.

7. Arzneimittel nach Anspruch 4, **dadurch gekennzeichnet, daß** die Teilchen eine feste Umhüllung aufweisen, in deren Inneren sich das LH gegebenenfalls zusammen mit dem FSH und pharmazeutischen Hilfsmittel befindet.

8. Arzneimittel nach Anspruch 7, **dadurch gekennzeichnet, daß** die Hülle ausgehend von biologisch abbaubaren Polymeren, wie Polymilchsäure oder ein Kopolymer von Milchsäure und Glykolsäure gebildet ist.

9. Arzneimittel nach Anspruch 8, **dadurch gekennzeichnet, daß** die Teilchen aus einem kontinuierlichen Netz von Trägermaterial bestehen, in dem das LH gegebenenfalls zusammen mit dem FSH dispergiert ist.
